(19) Europäisches Patentamt
European Patent Office
Office européen des brevets

(11) EP 1 335 756 B1

(12) # EUROPÄISCHE PATENTSCHRIFT

(45) Veröffentlichungstag und Bekanntmachung des Hinweises auf die Patenterteilung:
**31.05.2006 Patentblatt 2006/22**

(21) Anmeldenummer: **01988622.5**

(22) Anmeldetag: **25.10.2001**

(51) Int Cl.:
***A61L 15/60*** *(2006.01)* ***B01J 20/26*** *(2006.01)*
***C08F 8/30*** *(2006.01)*

(86) Internationale Anmeldenummer:
**PCT/EP2001/012315**

(87) Internationale Veröffentlichungsnummer:
**WO 2002/034384 (02.05.2002 Gazette 2002/18)**

(54) **HOCHQUELLBARE ABSORPTIONSMITTEL MIT EINER VERMINDERTEN TENDENZ ZUM VERBACKEN**

ABSORPTION AGENTS WITH A HIGH SWELLING CAPACITY, WITH A REDUCED TENDENCY TO CAKE

AGENT D'ABSORPTION HAUTEMENT GONFLABLE ET A FAIBLE TENDANCE A S'AGGLOMERER

(84) Benannte Vertragsstaaten:
**AT BE CH CY DE DK ES FI FR GB GR IE IT LI LU MC NL PT SE TR**

(30) Priorität: **25.10.2000 DE 10052966**

(43) Veröffentlichungstag der Anmeldung:
**20.08.2003 Patentblatt 2003/34**

(73) Patentinhaber: **Stockhausen GmbH**
**47805 Krefeld (DE)**

(72) Erfinder:
- **JONAS, Gerd**
**47807 Krefeld (DE)**
- **MERTENS, Richard**
**47803 Krefeld (DE)**
- **FRANK, Markus**
**47918 Tönisvorst (DE)**
- **DE MARCO, Michael**
**69469 Weinheim (DE)**

(74) Vertreter: **Herzog, Martin**
**Kahlhöfer . Neumann . Herzog . Fiesser,**
**Karlstrasse 76**
**40210 Düsseldorf (DE)**

(56) Entgegenhaltungen:
**EP-A- 0 467 073**
**EP-A- 0 509 708**
**WO-A-00/10619**
**WO-A-99/47249**
**DE-A- 10 016 041**
**DE-A- 19 801 933**
**US-A- 5 728 742**
**US-A- 5 945 495**
**US-A- 6 031 147**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 335 756 B1

**Beschreibung**

Die vorliegende Erfindung betrifft hochquellbare Absorptionsmittel mit verminderter Tendenz zum Verbacken an feuchter Umgebung und/oder hoher Temperaturen. Die vorliegende Erfindung betrifft desweiteren die Herstellung und Verwendung dieser Absorptionsmittel in Hygieneartikeln und in technischen Bereichen.

Wässrige Flüssigkeiten absorbierende Polymerisate, sogenannte Superabsorber sind durch zahlreiche Veröffentlichungen bekannt. Es kommen modifizierte natürliche Polymere und teilweise oder vollständig synthetische Polymere zum Einsatz. Die vollsynthetischen Polymeren werden in der Regel durch radikalische Polymerisation verschiedener hydrophiler Monomere in wässriger Lösung nach unterschiedlichen Methoden hergestellt. Im allgemeinen werden dabei Vernetzer mit einpolymerisiert, wodurch die Polymerisate nicht mehr wasserlöslich, sondern nur noch wasserquellbar sind. Als Superabsorber können z.B. Polymerisate auf Basis von (Meth-) Acrylsäure verwendet werden, die in teilweise neutralisierter Form als Alkalisalz vorliegen.

Superabsorbierende Polymerisate werden meist in Form von Granulaten als absorbierende Komponente in vielen sanitären Artikeln wie Windeln, Damenbinden oder medizinischen Saugeinlagen verwendet. Für die Herstellung dieser Artikel ist eine genaue Dosierung erforderlich, die nur durch eine konstante Förderung in den Produktionsanlagen gewährleistet werden kann. Problematisch für eine solche konstante Förderung wirkt sich die hohe Hygroskopizität der superabsorbierenden Polymeren aus. Diese Hygroskopizität führt zu einem Verbacken der Polymerpartikel, insbesondere bei hoher relativer Luftfeuchtigkeit und/oder bei hohen Temperaturen. Agglomerierte Superabsorber lassen sich nur ungenau dosieren und setzen sich an den Wänden der Produktionsanlagen ab, wodurch ein erhöhter Reinigungsaufwand resultiert. Daher hat es in der Vergangenheit nicht an Versuchen gefehlt, superabsorbierende Polymere mit geringerer Tendenz zum Verbacken zu entwickeln.

In einer Reihe von bekannten Verfahren wird eine Reduzierung der Hygroskopizität durch Aufbringen sehr feinteiliger anorganischer Pulver auf die Oberfläche der Polymerpartikel erreicht. So wird in der EP 0 388 120 A die Oberflächenbehandlung von Polymeren mit Siliciumdioxidpulver mit einer mittleren Teilchengröße von 0,1 bis 30 μm beschrieben.

In der US 4,734,478 werden Polymere offenbart, die nach der Polymerisation zunächst mit einer Mischung aus einem Polyalkohol und einem hydrophilen organischen Lösungsmittels versetzt und bei Temperaturen von > 90°C wärmebehandelt werden. Anschließend werden die nachvernetzten Polymeren mit 0.01 bis 10 Gew.% Silikastaub mit einem Partikeldurchmesser von weniger als 10 μm behandelt. Diese Polymere sollen außer einer geringeren Tendenz zum Verbacken auch eine hohe Wasseraufnahmekapazität aufweisen.

In der US 4,286,082 werden Verfahren zur Herstellung von wasserabsorbierenden Harzen offenbart, bei denen der Monomerenlösung wenigstens ein wasserlösliches, oberflächenaktives Reagenz zugesetzt wird und das erhaltene Polymere bei Temperaturen von 100 bis 230°C wännebehandelt wird. Die oberflächenaktiven Reagenzien sind vorzugsweise nichtionische Tenside, die einen HLB-Wert von 7 bis 20 aufweisen. Um die Verbackungstendenz dieser Polymere zu verringern, werden die Polymere mit ultramikroskopischen Silika vermischt.

Da durch die Behandlung der Polymere mit anorganischem Pulver deren Staubanteil erhöht wird, treten insbesondere bei mechanischer Belastung, wie im Rahmen einer pneumatischen Förderung, und dem daraus resultierenden Abrieb Staubprobleme auf. Diese Staubfreisetzung möchte man wegen deren Gesundheitsschädlichkeit vermeiden, so dass diese Polymere bei ihrer Herstellung und Verwendung schlechter handhabbar sind.

Versuche, Polymere mit geringen Staubanteile zur Verfügung zu stellen, sind in der US 5,994,440 beschrieben. Solche Polymere sollen durch Oberflächenbeschichtung von wasserabsorbierenden, vernetzten Polymerisaten mit hydrophilen, organischen Verbindungen, die nicht in die innere Struktur der Polymere eindringen, erhältlich sein. Geeignete organische Verbindungen sind aliphatische Polyole mit einem Molekulargewicht von mehr als 200 g/mol. Durch die Oberflächenbeschichtung soll erreicht werden, dass der Polymerstaub an den Polymerpartikeln bzw. an den Wänden der Aufbewahrungsbehälter anhaftet, so dass ein Stauben vermieden werden kann. Der lose Staubanteil dieser Polymere soll bei ≤2,5 ppm liegen, wobei hierzu Staubpartikel mit einem Durchmesser von ≤ 10 μm gezählt werden.

In einer Reihe anderer bekannter Verfahren wird die Oberfläche der absorbierenden Polymeren mit hydrophoben Agentien behandelt, um eine Reduzierung der Hygroskopizität zu erreichen. So werden in der EP 0 755 964 A2 hochquellfähige Hydrogele beschrieben, deren Oberfläche mit Wachsen beschichtet wurde. Hierzu können alle Wachse verwendet werden, die keine reaktiven Gruppen aufweisen, die mit den Carboxylgruppen der Polymeroberflächen reagieren können. Vorzugsweise werden Wachse mit einem Schmelzbereich von 30 und 180°C eingesetzt.

Die EP 0 509 708 A1 offenbart Polymere, die durch Oberflächenvernetzung mit Polyhydroxyverbindungen und durch Oberflächenbeschichtung mit einen HLB Wert zwischen 3 und 10 aufweisenden Tensiden erhältlich sind. Als Polyhydroxyverbindungen können alle Verbindungen eingesetzt werden, die wenigstens zwei Hydroxylgruppen aufweisen und die mit den Carboxylgruppen der Polymerpartikel reagieren können. Bevorzugte Polyhydroxyverbindungen sind u.a. Polyglykole oder niedere Glykolderivate. Als Tenside können insbesondere Sorbitanfettsäureester, ethoxylierte Sorbitanfettsäureester, Glycerin- oder Polyglycerinfettsäureester oder modifizierte, oberflächenaktive Polyester eingesetzt werden.

Nachteilig wirkt sich bei diesen Verfahren der Oberflächenbeschichtung von Polymeren mit hydrophoben Substanzen aus, dass sich die Hvdrophilie der Poly-meroberflächen verringert, wodurch zumindest erniedrigte Flüssigkeits-Aufnahmegeschwindigkeiten resultieren.

In der US 5,728,742 wird eine nicht agglomerierende, nicht staubende Zusammensetzung offenbart, die durch Nachbehandlung wasserabsorbierender, geringfügig vernetzter Polymere mit einem And-Agglommtionsmittel und einem hydrophilen Anti-Staub-Mittel erhalten werden. Diese Anti-Staub-Mittet sind entweder Polyole mit einem Molekulargewicht von größer 200 g/mol oder Polyalkylenglykole mit einem Molekulargewicht von 400 bis 6.000 g/mol. Als Anti-Agglomerationsmittel werden kationische Tenside, beispielsweise quaternäre Ammonium- oder Phosphonium-Salze eingesetzt.

EP 0 467 073 A beschreibt ein Verfahren zur Herstellung eines wasserunlöslichen, wasserabsorbierenden Harzes, bei dem eine wässrige Monomerlösung in einer Polymerisationswirichtung, welche in der Lage ist, die mit der wässrigen Lösung in Kontakt tretenden Flächen zu kühlen und zu erwärmen, ohne Rühren einer Polymerisation bei konstanter Temperatur unterworfen wird. Die durch dieses Verfahren erhaltenen Harze können unter anderem mit Kokussnußölfettsäurediethanolamid beschichtet und erhitzt werden.

Aufgabe der vorliegenden Erfindung war es daher, superabsorbierende Polymerisate zur Verfügung zu stellen, die gegenüber den nach dem Stand der Technik bekannten Absorptionsmitteln eine verminderte Tendenz zur Verbakkung, insbesondere bei feuchter Umgebung wie hoher Luftfeuchtigkeit und/oder hohen Temperaturen, bei im übrigen Zumindest gleichwertigen Produkteigenschaften, insbesondere wenigstens unveränderter Wasseraufnahmekapazität, Retention und Aufnahmegeschwindigkeit von Wasser oder wässrigen Flüssigkeiten, insbesondere Körperflüssigkeiten, aufweisen.

Die Aufgabe wird erfindungsgemäß durch das zur Verfügung stellen eines hochquellfähigen Absorptionsmittels mit einer verminderten Tendenz zum Verbacken an feuchter Umgebung und/oder bei hohen Temperaturen mindestens basierend auf Komponenten

1 einem mit Säuregruppen modifizierten, Wasser oder wässrige Flüssigkeiten absorbierenden natürlichen Polymeren oder einem wasserunlöslichen. an der Oberfläche nachvernetzten, Wasser oder wässrige Flüssigkeiten absorbierenden, vernetzten Polymerisat basierend auf polymerisierten, mindestens teilneutralisierten Säuregruppen enthaltenden Monomeren, das mit

II wenigstens einem Beschichtungsmittel aus der Gruppe Stickstoff- haltiger, nichtionischer Tenside behandelt

und
die Mischung aus der Komponente I und der Komponente II
einer Wärmebehandlung unterworfen worden ist.

Es ist in dem erfindungsgemäßen Absorptionsmittel bevorzugt, dass als Tenside wenigstens eine Verbindung der allgemeinen Formel I

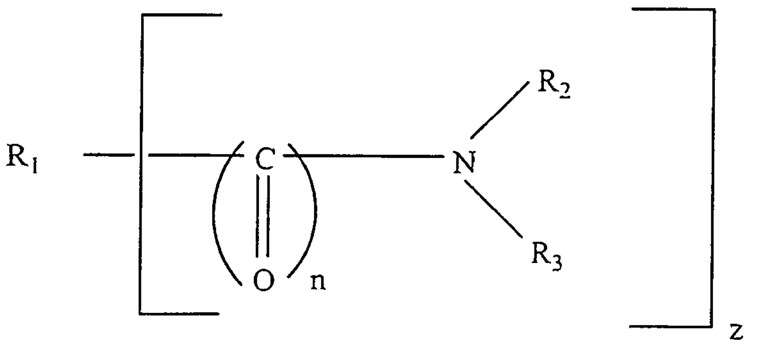

I

in der
$R_1$ einen z-fach substituierten, aliphatischen Rest, vorzugsweise einen z-fach substituierten, gesättigten oder ungesättigten, verzweigten oder unverzweigten aliphatischen Kohlenwasserstoffrest mit $C_1$ bis $C_{30}$, vorzugsweise $C_8$ bis $C_{12}$, der ggf. Arylreste, vorzugsweise einen Phenylrest, aufweisen kann, einen z-fach substituierten Benzolrest, der ggf. mit fünf- oder sechsgliedrigen, ggf. Heteroatome wie Sauerstoff, Phosphor oder Stickstoff, enthaltenden Ring kondensiert

sein kann,
bedeutet,
$R_2$
ein Wasserstoff;
einen aliphatischen Rest, vorzugsweise einen gesättigten oder ungesättigten, verzweigten oder unverzweigten Kohlenwasserstoffrest mit 2 bis 24 C-Atomen, vorzugsweise mit 8 bis 22 C-Atomen;
einen Hydroxyalkylenrest,
dessen Hydroxylgruppe vorzugsweise endständig ist und/oder ggf. mit 1 bis 50, vorzugsweise 1 bis 20, besonders bevorzugt 1 bis 10 Alkylenoxideinheiten, vorzugsweise Ethylen- und/oder Propylenoxideinheiten, alkoxyliert und/oder ggf. mit einer Carbonsäure, vorzugsweise mit einer $C_1$- bis $C_8$-Carbonsäure, verestert ist und
der 1 bis 8 C-Atome, vorzugsweise 1 bis 4 C-Atome im Alkylenrest aufweist;
oder einen N,N Dihydroxyalkylen-amino-alkylen-Rest mit jeweils 1 bis 4 C-Atomen im Alkylenrest
bedeutet,
$R_3$, gleich oder verschieden, die Bedeutung von $R_2$ hat, mit der Bedingung bei Amidverbindungen, dass wenigstens einer der Reste $R_2$ oder $R_3$ für einen Hydroxyalkylenrest oder einen alkoxylierten Hydroxyalkylenrest oder einen entsprechend veresterten, ggf. alkoxylierten Hydroxyalkylenrest mit der unter $R_2$ angegebenen Definition steht,
n 0 oder 1, vorzugsweise 1 bedeutet
und z für eine ganze Zahl von 1 bis 4 steht,
eingesetzt worden ist.

Vorzugsweise liegt die Komponente I als Pulver vor. Die Teilchengrößen dieses Pulvers liegt vorzugsweise zu mindestens 20 Gew.-%, vorzugsweise zu mindestens 50 Gew.-% und besonders bevorzugt zu mindestens 70 Gew.-% in einem Bereich von 150 bis 1000 μm. Es ist ferner bevorzugt, dass weniger als 20 Gew.-% und bevorzugt weniger als 10 Gew.-% der Teilchengrößen des Pulvers kleiner 150 μm sind. Die Gew.-%-Angaben dieses Absatzes beziehen sich immer auf das gesamte Pulver. Die Teilchengröße lässt sich nach ERT-420.1-99 bestimmen.

Überraschend wurde gefunden, dass durch Beschichtung von Wasser oder wässrige Flüssigkeiten absorbierende Polymeren mit wenigstens, einem erfindungsgemäßen Beschichtungsmittel II ggf. in Kombination mit einer Lewissäure III Absorptionsmittel mit einer signifikant reduzierten Tendenz zum Verbacken erhalten werden, während die übrigen anwendungstechnischen Eigenschaften, insbesondere Retention und Absorption unter Druck, nicht beeinträchtigt werden. Dabei wird auch eine verminderte Staubentwicklung der behandelten Polymere beobachtet.

Das erfindungsgemäße Absorptionsmittel weist mindestens eine, vorzugsweise alle, der nachfolgenden Eigenschaften auf:

(a) kein Verbacken nach mindestens 3, vorzugsweise mindestens 6 und besonders bevorzugt mindestens 24 h, darüber hinaus bevorzugt im Bereich von 3 bis 30 Stunden, Wärmebehandlung gemäß des nachstehend beschriebenen Anticaking Tests,

(b) eine Retention von mindestens 20 g/g, bevorzugt mindestens 25 g/g und besonders bevorzugt mindestens 30 g/g sowie darüber hinaus bevorzugt im Bereich von 20 bis 100 g/g,

(c) Einen Absortion under Load bei einer Belastung von 0,9 psi ($AUL_{0.9psi}$) von mindestens 7 g/g, vorzugsweise mindestens 15 g/g und besonders bevorzugt mindestens 20 g/g sowie besonders bevorzugt im Bereich von 7 bis 40 g/g.

Die sich aus den vorstehenden Eigenschaften ergebenden Eigenschaftskombinationen von zwei oder mehr dieser Eigenschaften stellen jeweils bevorzugte Ausführungsfonnen des erfindungsgemäßen Absorbtionsmittels dar, hierunter sind die Eigenschaftskombinationen ab, ac, bc bevorzugt, wobei ab und ac besonders bevorzugt ist.

Als wasserquellbare hydrophile Polymerisate kommen neben natürlichen, teilsynthetische oder vollsynthetische Polymerisate in Betracht. Als natürliche mit Säuregruppen, vorzugsweise Carboxylgruppen modifizierte Polymere können Carboxylgruppen aufweisende Polysaccharide, vorzugsweise Stärken, Cellulosen, Guar, wie z. B. Carboxymethylguar, Xanthane, Alginate, Gummi arabicum, Carboxymethylcellulose, Carboxymethylstärke und Mischungen dieser Polysaccharide zum Einsatz kommen. Diese Polymere sind wasserquellbar und teilweise bis vollständig wasserunlöslich.

Bevorzugt sind teilsynthetische und vollsynthetische Polymerisate, insbesondere anionische Polymerisate auf Basis (Meth)Acrylsäure, die in teilneutralisierter Form als Alkalisalze, insbesondere Natrium- und/oder Kaliumsalze, vorliegen. Der Neutralisationsgrad der sauren Monomerkomponenten kann schwanken, liegt aber vorzugsweise zwischen 25 und 85 Mol.%. Es kann sich um Homo- und Copolymerisate handeln, die allein aus Acrylsäure und/oder Methacrylsäure erhältlich sind, aus diesen Monomeren zusammen mit einem oder mehreren anderen Monomeren oder allein aus einem oder mehreren anderen Monomeren, aber beispielsweise auch um aufgepfropfte anionische Polymerisate, z.B. auf Basis (Meth)Acrylsäure, in teilneutralisierter Form als Alkalisalz vorliegend, z.B. um Pfropfpolymerisate

auf Polyvinylalkohol, Polysacchariden wie etwa Stärke oder Cellulose oder Derivaten hiervon oder auf Polyalkylenoxiden, wie Polyethylenoxiden oder Polypropylenoxiden.

Als Beispiele für Monomere, die neben (Meth)Acrylsäure bei der Herstellung der Polymerisate Verwendung finden können, seien Methyl-, Ethyl-, und -(Poly)Hydroxyalkylestei- der (Meth)Acrylsäure, (Meth)Acrylamid, Crotonsäure, Malein- und Fumarsäure, Itaconsäure, 2-Acrylamido-2-methylpropansulfonsäure, Vinylsulfonsäure und Vinylphosphonsäure und die Methyl-, Ethyl- und (Poly)Hydroxyalkylester und Amide dieser Säuren, amino- und ammoniumgruppenhaltige Ester und Amide aller genannten Säuren und wasserlösliche N-Vinylamide genannt, aber auch aus allen weiteren, bei der Herstellung von superabsorbierenden Polymerisaten üblichen Monomeren stammende Baueinheiten können im Polymerisat enthalten sein. Die Polymerisate sind bevorzugt vernetzt. Als Beispiele für geeignete, bei der Herstellung der absorbierenden Polymerisate einsetzbare, zwei oder mehr reaktive Gruppen enthaltende Vernetzerverbindungensind Polyglycycidylverbindungen wie Polyglycidylether, Methylenbis(meth)acrylamid, Bisacrylamidoessigsäure, Ester ungesättigter Säuren von Polyolen bzw. alkoxylierten Polyolen, z.B. Ethylenglykoldi(meth)acrylat oder Trimethylolpropantri(meth)acrylat oder Allylverbindungen, wie z.B. Allyl(meth)acrylat, Polyallylester, Tetraallyloxyethan, Triallylamin, Tetraallylethylendiamin oder Allylester der Phosphorsäure sowie Vinylphosphonsäurederivate bzw. deren Mischungen geeignet. Der Anteil an Vernetzern, die bereits bei der Herstellung der absorbierenden Polymerisate zugegeben sind, liegt bevorzugt bei 0,01 bis 20 Gew.%, besonders bevorzugt bei 0,1 bis 3 Gew.%, bezogen auf die eingesetzten Gesamtmenge an Monomeren.

Die Herstellung der Polymerisate erfolgt nach an sich bekannten Methoden, wie sie z.B. in der DE 40 20 780 C1 zusammengestellt sind. Bevorzugt erfolgt die Herstellung durch Polymerisation in wässriger Lösung nach dem Verfahren der sogenannten Gelpolymerisation.

Das Polymerisatpulver, das durch Zerkleinerung, Trocknung und Mahlung des Polymerisatgele erhalten wird, kann einer Oberflächennachvernetzung unterworfen werden.

Vor der Oberflächennachvernetzung wird das Polymerisat vorzugsweise getrocknet, gemahlen und auf die für die jeweils anwendungstechnisch günstige Kornfraktion abgesiebt und dann der Oberflächennachvernetzungsreaktion zugeführt.

In manchen Fällen hat es sich jedoch auch bewährt, die Oberflächennachvernetzer bereits vor der Trocknung des Polymergels bzw. vor der Zerkleinerung des teilweise oder überwiegend getrockneten Polymers zuzufügen. Eine erfindungsgemäß durchzuführende Oberflächennachvernetzung wird z.B. in der US 4 666 983 und der DE 40 20 780 beschrieben. Der Zusatz der Oberflächennachvernetzungsmittel erfolgt häufig vorteilhafterweise auch in Form einer Lösung in Wasser, organischen Lösemitteln oder deren Mischungen, insbesondere dann, wenn geringe Mengen an Oberflächennachvernetzungsmittel angewandt werden. Geeignete Mischaggregate zum Aufbringen des Oberflächennachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer, sowie kontinuierlich arbeitende senkrechte Mischer in denen das Pulver mittels rotierender Messer in schneller Frequenz gemischt wird (Schugi-Mischer). Nachdem das Oberflächennachvernetzungsmittel mit dem Polymerisat vermischt worden ist, wird zur Durchführung der Oberflächennachvernetzungsreaktion auf Temperaturen von 60 bis 250 °C, bevorzugt auf 135 bis 200°C und besonders bevorzugt auf 150 bis 185°C erhitzt. Die Zeitdauer der Nacherhitzung ist dadurch begrenzt, dass das gewünschte Eigenschaftsprofil des Polymerisates infolge von Hitzeschädigung nicht beinträchtigt wird.

Als Nachvernetzungsmittel zur Oberflächennachvernetzung der Polymerisate werden vorzugsweise organische Nachvernetzer, d. h. mit den auf der Oberfläche vorhandenen COOH-Gruppen des Polymerisats reagierende Verbindungen, wie Alkylencarbonate, z. B. 1,3-Dioxolan-2-on, 4-Methyl-1,3-dioxolan-2-on, 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on oder 1,3-Dioxepan-1-on eingesetzt. Besonders bevorzugt sind 1,3-Dioxolan-2-on und 4-Methyl-1,3-dioxolan-2-on.

Weiterhin können als Oberflächennachvernetzungsmittel folgende Verbindungen eingesetzt werden: Polyhydroxyverbindungen wie zum Beispiel Ethylenglykol, Propylenglykol, Diethylenglykol, Dipropylenglykol, Triethylenglykol, Tetraethylenglykol, Tetrapropylenglykol, Polyethylenalykol, Polypropylenglykol, 1,3-Propandiol, Glycerin, Polyglycerin, 1,4-Butandiol, 1,5-Pentandiol, 1,6-Hexandiol, Trimethylolpropan, Pentaerythrit oder Sorbitol; Aminoalkohole wie z.B. Diethanolamin, Triethanolamin. Weitere, aufgrund ihres toxikologischen Potentials aber als kritisch einzustufende und deshalb auch nicht zu bevorzugende organischen Oberflächenvernetzungsmittel sind: Polyepoxide wie z.B. Ethylenglyloldiglycidylether, Polyethylenglyloldiglycidylether, Glycerolpolyglycidylether Polyglycerolpolyglycidylether, Propylengylcoldiglycidylether, Polypropylenglyloldiglycidylether, Glycidol; Polyisocyanate wie beispielsweise 2,4-Toluoldiisocyanat und Hexamethylendiisocyanat; Halogenepoxide wie beispielsweise Epichlor- und E-pibromhydrin und $\alpha$-Methylepichlorhydrin; Polyaminverbindungen wie beispielsweise Ethylendiamin, Diethylentriamin, Triethylentetramin, Polyallylamin oder Polyethylenimin. Ferner sind als Oberflächenvernetzungsmittel Polyoxazolinverbindungen wie beispielsweise 1,2-Ethylenbisoxazolin einsetzbar. Das organische Nachvernetzungsmittel wird vorzugsweise in Mengen von 0,01 - 5 Gew.%, besonders bevorzugt von 0,1 - 2,5 Gew.% und ganz besonders bevorzugt von 0,1 bis 1 Gew.% jeweils bezogen auf das Polymerisat eingesetzt.

Als Beschichtungsmittel II werden nichtionische, stickstoffhaltige Tenside, vorzugsweise Verbindungen der allgemeinen Formel I eingesetzt. Es kann aber auch eine Mischung aus wenigstens zwei Verbindungen dieser Formel eingesetzt werden. Vorzugsweise leitet sich das Beschichtungsmittel II von einer Fettsäure wie Caprylsäure, Caprinsäure, Laurinsäure, Myristinsäure, Palmitinsäure, Palmitoleinsäure, Magarinsäure, Stearinsäure, Ölsäure, Linolsäure, Linolensäure, Arachin, Eurecasäure ab.

Insbesondere werden als Beschichtungsmittel II Fettsäurealkanolamide, die entsprechenden ethoxylierten und/oder proproxylierten Verbindungen, die jeweils veresterten Verbindungen oder Fettsäureamine, die ethoxylierten und/oder propoxylierten entsprechenden Verbindungen, die jeweils auch verestert sein können, eingesetzt. Darunter sind beispielsweise zu verstehen Laurinsäuremonoethanolamid, Kokosfetisäuremonoethanolamid, Stearinsäuremonoethanolamid, Rizinolsäuremonoethanolamid, Undecylensäuremonoethanolamid, Laurinsäurediethanolamid, Kokosfettsäurediethanolamid, Sojafettsäurediethanolamid, Linolsäurediethanolamid, Laurinmyristinsäurediethanolamid, Ölsäurediethanolamid, Laurinsäureisopropanolamid, Kokosfettsäureisopropanolamid, Ölsäureisopropanolamid, Undecylensäurepolydiethanolamid, Kokosfettsäurepolydiethanolamid, Talgamin, Talgpropylendiamin, Kokosfettsäureamin, Laurylamin, Oleylamin, Stearylamin, Talgfettamin, die Ethoxylate und/oder Propoxylate der genannten Verbindungen, die mit 1 bis 50, bevorzugt 1 bis 20, Alkylenoxid-Einheiten aufweisen können, sowie Ester der Verbindungen wie beispielsweise Kokosfettsäuremonoethanolamidacetat. Beliebige Gemische dieser Verbindungen können auch eingesetzt werden.

Die Beschichtungsmittel II werden vorzugsweise in einer Konzentration von 50 bis 50.000 ppm, besonders bevorzugt 100 bis 5.000 ppm und ganz besonders bevorzugt 300 bis 3.000 ppm, bezogen auf das Komponente I, eingesetzt.

In einer besonderen Ausführungsform der vorliegenden Erfindung werden die Polymere neben wenigstens einem Beschichtungsmittel II zusätzlich mit zumindest einer Lewissäure als Beschichtungsmittel III versetzt. Erfindungsgemäß können als Lewissäuren alle Elektronenpaarakzeptoren eingesetzt werden.

Die als Lewissäure III bzw. Beschichtungsmittel III in dem erfindungsgemäßen Absorptionsmittel einsetzbaren Verbindungen sind anorganische Säuren, wasserlösliche, gesättigte oder ungesättigte organische Säuren, wasserlösliche Hydroxycarbonsäuren oder wasserlösliche saure Salze.

Als Lewissäure werden vorzugsweise anorganische Säuren, wie Halogenwasserstoffe, Halogensauerstoffsäuren. Sauerstoffsäuren des Schwefels oder des Selens, Sauerstoffsäuren des Stickstoff oder des Phosphors, organische Säuren, wie wasserlösliche gesättigte oder ungesättigte organische Säuren, und/oder wasserlösliche saure Salze, wie wasserlösliche Bromide, Chloride, Nitrate, Sulfate, Phosphate oder Salze von organischen Säuren wie Acetate, Formiate, Oxalate und Lactate von beispielsweise den Metallen Al, Fe, Zn, Sb, As, Sn, Cu, Mg, Ca, Cr, Ga, V, Ti, Bi, Tl, In, Mn, Ni, Co, Be und Zirkonium, eingesetzt.

Bevorzugt werden als anorganische Säuren Salzsäure, Perchlorsäure, Bromsäure, Bromwasserstoff, Schwefelsäure, schweflige Säure, Selensäure, Salpetersäure, Phosphonsäure oder Phosphorsäure, als organische wasserlösliche Säuren Carbonsäure, Hydroxycarbonsäuren, Sulfonsäuren oder Phosphonsäuren oder entsprechende Aminosäuren, z.B. Acrylsäure, Methacrylsäure, Ameisensäure, Essigsäure, Propionsäure, Buttersäure, Oxalsäure, Malonsäure, Bernsteinsäure, Milchsäure, Maleinsäure, Fumarsäure, Benzoesäure, Phthalsäure, Salicylsäure, Weinsäure, Citronensäure, p-, m- und o-Toluolsulfonsäure, Benzolsulfonsäure, Aminomethansulfonsäure, Aminomethanphosphonsäure, und als saure Salze Aluminiumsalze, Alaune und deren unterschiedliche Hydrate, wie $AlCl_3$ x 6 $H_2O$, $NaAl(SO_4)_2$ x 12 $H_2O$, $KAl(SO_4)_2$ x 12 $H_2O$ oder $Al_2(SO_4)_3$ x 14-18 $H_2O$, Zinksalze und deren Hydrate, wie $ZnCl_2$, $ZnSO_4$ x 1-7 $H_2O$ und $Zn(CH_3COO)_2$ x 2 $H_2O$, Eisensalze und deren Hydrate, wie $NaFe(SO_4)_2$ x 12 $H_2O$, $KFe(SO_4)_2$ x 12 $H_2O$ und $Fe_2(SO_4)_3$ x n $H_2O$, Mg-Salze wie $MgCl_2$ oder $MgSO_4$, Doppelsalze, aber auch Gemische der Salze und Gemische der anorganischen und/oder der organischen Säuren und Gemische der Salze mit den anorganischen und/oder den organischen Säuren eingesetzt.

Ganz besonders bevorzugt werden als anorganische Säuren Schwefelsäure oder Phosphorsäure, als saure Salze $AlCl_3$ x 6 $H_2O$, $Al_2(SO_4)_3$ x 14-18 $H_2O$, $ZnCl_2$, $ZnSO_4$ x 1-7 $H_2O$, Zn $(CH_3COO)_2$ x $2H_2O$, $MgSO_4$, $MgCl_2$, bzw. als organische Säuren Essigsäure, Oxalsäure, Milchsäure, Zitronensäure oder Weinsäure eingesetzt.

Erfindungsgemäß ist es besonders bevorzugt, dass als Lewissäure Schwefelsäure, Phosphorsäure, Ameisensäure, Essigsäure, Zitronensäure oder p-Toluolsulfonsäure, Aluminiumsalze oder Alaune und/oder deren unterschiedlichen Hydrate, Zinksalze und/oder deren Hydrate, Magnesiumsalze und/oder deren Hydrate und/oder Doppelsalze eingesetzt worden sind.

Erfindungsgemäß wird wenigstens eine Lewissäure eingesetzt. Es kann aber auch eine Mischung aus wenigstens zwei der genannten Lewissäuren verwendet werden.

Die Gesamtmenge an Beschichtungsmittel II und III soll 100 bis 50.000 ppm, bevorzugt von 300 bis 25.000 ppm und besonders bevorzugt von 500 bis 13.000 ppm, bezogen auf das Komponente I, betragen.

Das Beschichtungsmittel II kann wahlweise mit der Lewissäure III, wahlweise vor oder nach der Durchführung des Oberflächennachvernetzungsschrittes, aber auch gemeinsam mit dem Nachvernetzungsmittel aufgebracht und auf dem Polymerisat 1 einer Wärmebehandlung unterworfen werden. Alternativ kann bei einer Kombination des Beschichtungsmittels II mit der Lewissäure III ein getrenntes Aufbringen der beiden Verbindungen, vorzugsweise als wässrige

Lösungen, oder ein gemeinsames Aufbringen auf das Polymerisat I erfolgen, ggf. gemeinsam mit dem Nachvernetzungsmittel, bevorzugt als eine wässrige Lösung. Bevorzugt wird das Beschichtungsmittel II ggf, in Kombination mit der Lewissäure III gemeinsam mit dem Nachvernetzungsmittel aufgebracht und das beschichtete Polymerisat anschließend einer Wärmebehandlung unterworfen, da dadurch ein zusätzlicher Prozeßschritt eingespart werden kann. Es ist besonders bevorzugt, dass die Komponente I mit einer wässrigen Lösung der Beschichtungsmittel II und III beschichtet und zur Reaktion gebracht worden ist. Es ist ferner bevorzugt, dass als Beschichtungsmittel II ein Fettsäurealkanolamid oder Fettsäureamin gemäß der Formel I, dass gegebenenfalls alkoxyliert und/oder verestert ist, eingesetzt worden ist.

Geeignete Lösungsmittel sind Wasser oder polare, mit Wasser mischbare organische Lösungsmittel wie beispielsweise Aceton, Methanol, Ethanol oder 2-Propanol bzw. deren Gemische, bevorzugt wird Wasser verwendet. Der Begriff wässrige Lösung im Sinne der Erfindung bedeutet in Bezug auf die Lösungsmittelkomponente, dass neben dem Wasser auch noch organische Lösungsmittel enthalten sein können. Die Konzentration des ggf. vorhandenen Nachvernetzungsmittels in der Beschichtungslösung kann in weiten Grenzen schwanken und liegt meistens im Bereich von 1 bis 80 Gew.%, vorzugsweise in einem Bereich von 1 bis 60 Gew.% und ganz besonders bevorzugt in einem Bereich von 10 bis 50 Gew.%. Die Konzentration des Beschichtungsmittel II oder gegebenenfalls der Lewissäure III in der Lösung kann ebenfalls in weiten Grenzen schwanken und liegt vorzugsweise in einem Bereich von 0,5 bis 80 Gew.%, vorzugsweise in einem Bereich von 0,5 bis 60 Gew.% und besonders bevorzugt in einem Bereich zwischen 0,5 bis 30 Gew.%. Das bevorzugte Lösungsmittel für das ggf. vorhandene organische Nachvernetzungsmittel und das Beschichtungsmittel II und gegebenenfalls der Lewissäure III ist Wasser, das vorzugsweise in einer Menge von 0,5-10 Gew.%, besonders bevorzugt von 0,5 - 5 Gew.% und ganz besonders bevorzugt von 0,5 - 4 Gew.%, bezogen auf das Polymerisat I, verwendet wird.

Um die gewünschten Eigenschaften zu erzielen, ist eine gleichmäßige Verteilung der Beschichtungslösung (en) auf dem absorbierenden Polymerisat erforderlich. Dazu führt man die innige, vorzugsweise homogene Vermischung der Komponenten in geeigneten Mischern, wie z.B. Wirbelbettmischer, Schaufelmischer, Walzenmischer oder Doppelschneckenmischer durch.

Es besteht auch die Möglichkeit, die Beschichtung des Polymerisates während der Herstellung des Polymerisats, vorzugsweise im Endstadium der Polymerisation vorzunehmen. Hierzu ist besondere die inverse Suspensionspolymerisation geeignet.

Die Wärmebehandlung der mit dem Beschichtungsmittel II bzw. einer entsprechenden Beschichtungslösung beschichteten Komponente I wird vorzugsweise bei Temperaturen von 100 und 250°C, besonders bevorzugt von 150 und 230°C, ganz besonders bevorzugt von 150 und 210°C durchgeführt, um die Komponente I mit dem Beschichtungsmittel zur Reaktion zu bringen.

Sofern Beschichtungsmittel II und III bzw. eine entsprechende Beschichtungslösung eingesetzt werden, erfolgt die Wärmebehandlung vorzugsweise bei Temperaturen von 40 bis 250°C, besonders bevorzugt von 100 bis 230°C, ganz besonders bevorzugt von 130 bis 210°C, wobei die Komponente I mit dem Beschichtungsmittel zur Reaktion gebracht wird.

Vorzugsweise wird das Beschichtungsmittel II in Kombination mit einer Lewissäure III auf das absorbierende Polymerisat wahlweise vor, nach oder gemeinsam mit dem Nachvernetzungsmittel vor der Wärmebehandlung aufgebracht, da in diesem Fall die Temperatur und Dauer der Wärmebehandlung niedriger und kürzer sein kann als bei der Behandlung des absorbierenden Polymerisats mit dem Beschichtungsmittel II ohne die Lewissäure III.

Die Teilchengröße des zu beschichtenden Pulvers liegt vorzugsweise in einem Bereich von 50 bis 5 000 $\mu$m, besonders bevorzugt 50 bis 1 000 $\mu$m und ganz besonders bevorzugt zwischen 50 bis 850 $\mu$m. Die Teilchengröße wird nach der bekannten Siebmethode bestimmt.

Die Dauer der Wärmebehandlung ist auch abhängig von der gewählten Temperatur. Dabei ist zu beachten, dass der Zeitbedarf um so geringer ist, je höher die gewählte Temperatur ist. Behandlungsdauer und -temperatur werden dabei so gewählt, dass die behandelten Polymerisate eine verminderte Tendenz zum Verbacken, d. h. den Anti-caking Test ($\geq$ 3 h) bestehen, unter Beibehaltung oder Verbesserung der Werte für die Retention, Absorption unter Druck und Aufnahmegeschwindigkeit für Wasser oder wässrige Flüssigkeiten, insbesondere Körperflüssigkeiten, von nicht erfindungsgemäß ausgerüsteten superabsorbierenden Polymerisaten. Für Polymerisate auf der Basis von teilneutralisierten und nachvernetzten Poly(meth)acrylsäuren bedeutet dies eine Retention von $\geq$ 20 g/g und einen $AUL_{0,9\ psi}$ von $\geq$ 19 g/g gemäß den nachfolgenden Methoden bestimmt.

Weiterhin betrifft die Erfindung Absorptionsmittel, die durch das erfindungsgemäße Verfahren erhältlich sind.

Die erfindungsgemäß behandelten Polymerisate zeichnen sich durch eine gute Verarbeitbarkeit, wie einer guten Förder- und Dosierbarkeit aus.

Die erfindungsgemäßen Polymerisate oder Absorptionsmittel können vorzugsweise in absorbierenden Hygieneprodukten wie Babywindeln, Inkontinenzprodukten für Erwachsene und Damenbinden eingesetzt.

Absorbierende Hygieneprodukte besitzen in der Regel einen allgemeinen Aufbau aus einer körperzugewandten, flüssigkeitsdurchlässigen Abdeckung, einer flüssigkeitsabsorbierenden Sauglage sowie einer im wesentlichen flüssigkeitsundurchlässigen, körperabgewandten Außenschicht. Optional finden auch weitere Konstruktionen zur schnellen

Aufnahme und Verteilung von Körperflüssigkeit im Saugkern Anwendung. Diese Konstruktionen werden häufig, aber nicht zwingend zwischen der körperzugewandten, flüssigkeitsdurchlässigen Abdeckung und der flüssigkeitsabsorbierenden Sauglage eingesetzt.

Die flüssigkeitsdurchlässige Abdeckung besteht in der Regel aus einem nichtgewebten, faserigen Vlies oder einer anderen porösen Konstruktion.

Als Materialien für diese Abdeckung kommen z. B. synthetische Polymere wie etwa Polyvinylchlorid oder -fluorid, Polytetrafluorethylen (PTFE), Polyvinylalkohole und -Derivate, Polyacrylate, Polyamide, Polyester, Polyurethane, Polystyrol, Polysiloxane oder Polyolefine (z.B. Polyethylen (PE) oder Polypropylen (PP)) sowie natürliche Fasermaterialien sowie beliebige Kombinationen aus den vorgenannten Materialien im Sinne von Mischmaterialien oder Verbundmaterialien oder Copolymerisaten in Frage.

Die flüssigkeitsdurchlässige Abdeckung hat hydrophilen Charakter. Sie kann zudem aus einer Kombination von hydrophilen und hydrophoben Bestandteilen bestehen. Bevorzugt ist in der Regel eine hydrophile Ausrüstung der flüssigkeitsdurchlässigen Abdeckung, um schnelle Einsickerzeiten von Körperflüssigkeit in die flüssigkeitsabsorbierende Sauglage zu ermöglichen, jedoch werden auch partiell hydrophobierte Abdeckungen verwendet.

Flüssigkeitsabsorbierende Sauglage

Die flüssigkeitsabsorbierende Sauglage enthält die superabsorbierenden erfindungsgemäßen Polymerisat Pulver bzw. Granulate und weitere Komponenten aus beispielsweise faserigen Materialien, schaumförmigen Materialien, filmbildenden Materialien oder porösen Materialien sowie Kombinationen von zwei oder mehreren dieser Materialien. Jedes dieser Materialien kann entweder natürlichen oder synthetischen Ursprungs sein oder durch chemische oder physikalische Modifikation von natürlichen Materialien hergestellt worden sein. Die Materialien können hydrophil oder hydrophob sein, wobei hydrophile Materialien bevorzugt sind. Dies gilt insbesondere für solche Zusammensetzungen, die ausgeschiedene Körperflüssigkeiten effizient aufnehmen und in Richtung zu weiter von der Eintrittsstelle der Körperflüssigkeit entfernte Regionen des absorbierenden Kerns transportieren sollen.

Als hydrophile Fasermaterialien sind geeignet z.B. Cellulosefasern, modifizierte Cellulosefasern (z.B. versteifte Cellulosefasern), Polyesterfasern (z.B. Dacron), hydrophiles Nylon oder aber auch hydrophilisierte hydrophobe Fasern, wie z. B. mit Tensiden hydrophilisierte Polyolefine (PE, PP), Polyester, Polyacrylate, Polyamide, Polystyrol, Polyurethane und andere.

Bevorzugt werden Cellulosefasern und modifizierte Cellulosefasern eingesetzt. Kombinationen von Cellulosefasern und/oder modifizierten Cellulosefasern mit synthetischen Fasern wie z. B. PE/PP Verbundmaterialien, sogenannte Bikomponentenfasern, wie sie z.B. zur Thermobondierung von Airlaidmaterialien verwendet werden oder anderen Materialien sind ebenfalls gebräuchlich.

Die Fasermaterialien können in verschiedenen Anwendungsformen vorliegen, z.B. als lose aus einem Luftstrom oder aus wässriger Phase abgeschiedene oder abgelegte Cellulosefasern, als nichtgewebtes Vlies oder als Tissue. Kombinationen verschiedener Anwendungsformen sind möglich.

Optional können neben den erfindungsgemäßen Superabsorbern weitere pulverförmige Substanzen eingesetzt werden, wie z.B. geruchsbindende Substanzen wie Cyclodextrine, Zeolithe, anorganische oder organische Salze und ähnliche Materialien.

Als poröse Materialien und schaumförmige Materialien können z.B. Polymerschäume eingesetzt werden, wie sie in den Schriften DE 44 18 319 A1 und DE 195 05 709 A1 beschrieben sind, die hiermit als Referenz eingeführt werden und somit als Teil der Offenbarung gelten.

Zur mechanischen Stabilisierung der flüssigkeitsabsorbierenden Sauglage können thermoplastische Fasern (z.B. Bikomponentenfasern aus Polyolefinen, Polyolefingranulate, Latexdispersionen oder Heisskleber) verwendet werden. Optional werden eine oder mehrere Lagen Tissue zur Stabilisierung verwendet.

Die flüssigkeitsabsorbierende Sauglage kann einlagig sein oder aus mehreren Schichten bestehen. Bevorzugt werden Konstruktionen verwendet, die aus hydrophilen Fasern, bevorzugt Cellulosefasern, optional einer Konstruktion zur schnellen Aufnahme und Verteilung von Körperflüssigkeit wie zum Beispiel chemisch versteifte (modifizierte) Cellulosefasern oder Highloftvliese aus hydrophilen oder hydrophilisierten Fasern sowie superabsorbierenden Polymerisate bestehen.

Das erfindungsgemäße superabsorbierende Polymerisate kann dabei homogen in den Cellulosefasern oder den versteiften Cellulosefasern verteilt sein, es kann lagig zwischen den Cellulosefasern oder den versteiften Cellulosefasern eingebracht sein, oder die Konzentration des superabsorbierenden Polymerisate kann innerhalb der Cellulosefasern oder versteiften Cellulosefasern einen Gradienten aufweisen. Das Verhältnis der Gesamtmenge an superabsorbierendem Polymer und der Gesamtmenge an Cellulosefasern oder den versteiften Cellulosefasern im absorbierenden Saugkern kann zwischen 0 zu 100 und 80 zu 20 variieren, wobei in einer Ausführungsform lokal, z. B. bei Gradienteneintrag oder schichtweisem Eintrag Konzentrationen von bis zu 100 % Polymerisat erreicht werden können. Derartige Konstruktionen mit Bereichen hoher Konzentrationen von absorbierendem Polymer, wobei der Anteil von Superabsorber in bestimmten Bereichen zwischen 60 und 100 Gew.-%, am stärksten bevorzugt zwischen 90 und 100 Gew.-%, jeweils bezogen auf die Konstruktion, liegt, sind beispielsweise auch in der Patentschrift US 5,669,894 beschrieben, die hiermit als Referenz eingeführt wird und somit als Teil der Offenbarung gilt.

Optional können auch mehrere verschiedene absorbierende Polymerisate, die sich zum Beispiel in der Sauggeschwindigkeit, der Permeabilität, der Speicherkapazität, der Absorption gegen Druck, der Kornverteilung oder auch der chemischen Zusammensetzung unterscheiden, gleichzeitig eingesetzt werden. Diese verschiedenen Polymerisate können miteinander vermischt in das Saugkissen eingebracht werden oder aber lokal differenziert im Absorbent Core plaziert werden. Eine solche differenzierte Plazierung kann in Richtung der Dicke des Saugkissens oder der Länge oder Breite des Saugkissens erfolgen.

In der flüssigkeitsabsorbierenden Sauglage befindet sich eine oder mehrere der oben beschriebenen, superabsorbierende Polymere enthaltenden Lagen aus Cellulosefasern oder versteiften Cellulosefasern. In einer bevorzugten Ausführungsform werden Konstruktionen aus Kombinationen von Lagen mit homogenem Superabsorbereintrag und zusätzlich schichtweiser Einbringung verwendet.

Optional werden diese erwähnten Strukturen auch durch weitere Lagen von reinen Cellulosefasern oder versteiften Cellulosefasern an der körperzugewandten Seite und / oder auch der körperabgewandten Seite ergänzt.

Die oben beschriebenen Konstruktionen können sich auch mehrfach wiederholen, wobei es sich um eine Aufeinanderschichtung zweier oder mehrerer gleicher Lagen oder aber auch um Aufeinanderschichtung zweier oder mehrerer unterschiedlicher Konstruktionen handeln kann. Dabei liegen die Unterschiede in wiederum rein konstruktiver Art oder aber im Typ des verwendeten Materials, wie z.B. die Verwendung von in den Eigenschaften differierender absorbierender Polymere oder aber verschiedener Zellstoffarten.

Optional sind das gesamte Saugkissen oder aber auch einzelne Lagen der flüssigkeitsabsorbierenden Sauglage durch Lagen von Tissue von anderen Komponenten getrennt oder stehen in direktem Kontakt mit anderen Lagen oder Komponenten.

Exemplarisch können zum Beispiel die Konstruktion zur schnellen Aufnahme und Verteilung von Körperflüssigkeit und die flüssigkeitsabsorbierende Sauglage durch Tissue voneinander getrennt sein oder aber in direktem Kontakt miteinander stehen. Sofern keine separate Konstruktion zur schnellen Aufnahme und Verteilung von Körperflüssigkeit zwischen der flüssigkeitsabsorbierenden Sauglage und der körperzugewandten flüssigkeitsdurchlässigen Abdeckung existiert, sondern der Effekt der Flüssigkeitsverteilung z.B durch die Verwendung einer speziellen körperzugewandten, flüssigkeitsdurchlässigen Abdeckung erreicht werden soll, kann die flüssigkeitsabsorbierende Sauglage ebenfalls optional von der körperzugewandten, flüssigkeitsdurchlässigen Abdeckung durch ein Tissue getrennt sein.

Statt Tissue kann optional auch nichtgewebtes Vlies in die flüssigkeitsabsorbierende Sauglage eingebracht werden. Beide Komponenten führen zu dem erwünschten Nebeneffekt der Stabilisierung und Festigung des Absorptionskerns im feuchten Zustand.

Herstellverfahren der flüssigkeitsabsorbierenden Sauglage

Faserhaltige, superabsorberhaltige, flüssigkeitsverteilende und -speichernde Schichten lassen lassen sich mit einer Vielzahl von Herstellverfahren generieren.

Neben den etablierten konventionellen Prozessen, wie Sie vom Fachmann allgemein unter Drumforming mit Hilfe von Formrädern, -taschen und Produktformen und entsprechend angepassten Dosiereinrichtungen für die Rohstoffe zusammengefasst werden, sind moderne etablierte Verfahren wie der Airlaidprozess (z.B. EP 850 615, Sp. 4 Zeile 39 bis Sp. 5 Zeile 29, US 4.640.810) mit allen Formen der Dosierung, Ablage der Fasern und Verfestigung wie Hydrogenbonding (z.B. DE 197 50 890, Sp. 1 Zeile 45 bis Sp. 3 Zeile 50, Thermobonding, Latexbonding (z.B. EP 850 615, Sp. 8 Zeile 33 bis Sp. 9 Zeile 17 und Hybridbonding, der Wetlaid Prozeß (z.B. PCT WO 99/49905, Sp. 4 Zeile 14 bis Sp. 7 Zeile 16), Carding-, Meltblown-, Spunblown- Prozesse sowie ähnliche Prozesse zur Herstellung von superabsorberhaltigen Non-Wovens (im Sinne der Definition der EDANA, Brüssel) auch in Kombinationen dieser Verfahren mit- und untereinander übliche Methoden zur Herstellung von den o.g. Flüssigkeitsspeichern. Die oben genannten Schriften werden als Referenz eingeführt und gelten somit als Teil der Offenbarung.

Als weitere Verfahren kommen die Herstellung von Laminaten im weitesteten Sinne sowie von extrudierten und coextrudierten, naß- und trocken- sowie nachträglich verfestigten Strukturen in Frage.

Eine Kombinationen dieser Verfahren mit- und untereinander ist ebenfalls möglich.

Konstruktionen zur schnellen Aufnahme und Verteilung von Körperflüssigkeit Eine Konstruktion zur schnellen Aufnahme und Verteilung von Körperflüssigkeit besteht zum Beispiel aus chemisch versteiften (modifizierten) Cellulosefasern oder Highloftvliesen aus hydrophilen oder hydrophilisierten Fasern oder einer Kombination von beidem.

Chemisch versteifte, modifizierte Cellulosefasern können zum Beispiel erzeugt werden aus Cellulosefasern, die durch Vernetzer wie z.B. $C_2$ - $C_8$ Dialdehyde, $C_2$ - $C_8$ Monoaldehyde mit einer zusätzlichen Säurefunktion oder $C_2$ - $C_9$ Polycarbonsäuren in einer chemischen Reaktion umgesetzt werden. Spezielle Beispiele sind: Glutaraldehyd, Glyoxal, Glyoxalsäure oder Zitronensäure. Ebenfalls bekannt sind kationisch modifizierte Stärke oder Polyamid-Epichlorhydrinharze (z. B. KYMENE 557H, Hercules Inc., Wilmington , Delaware). Durch die Vernetzung wird eine verdrehte, gekräuselte Struktur erreicht und stabilisiert, die sich vorteilhaft auf die Geschwindigkeit der Flüssigkeitsaufnahme auswirkt.

Flächengewicht und Dichte von absorbierenden Artikeln

Die absorbierenden Hygieneprodukte können in ihrem Flächengewicht und Dicke und damit der Dichte stark variieren. Typischerweise liegen die Dichten der Bereiche der Absorptionskerne zwischen 0,08 und 0,25 g/cm$^3$. Die Flächengewichte liegen zwischen 10 und 1000 g/m$^2$, wobei bevorzugt Flächengewichte zwischen 100 und 600 g/m$^2$ realisiert werden (siehe auch US 5,669,894, die hiermit als Referenz eingeführt wird und somit als Teil der Offenbarung gilt). Die Dichte variiert in der Regel über die Länge des absorbierenden Kerns. Dies tritt als Folge einer gezielten Dosierung der Cellulosefaser- oder versteiften Cellulosefasermenge oder der Menge des superabsorbierenden Polymers ein, da diese Komponenten in bevorzugten Ausführungsformen stärker in den Frontbereich des absorbierenden Einwegartikels eingebracht werden.

Diese gezielte Erhöhung des absorbierenden Materials in bestimmten Regionen des absorbierenden Kerns kann auf andere Weise auch z.B. durch Herstellung eines entsprechend großen, mittels eines airlaid- oder wetlaid-Verfahrens hergestellten Flächengebildes bestehend aus hydrophilen Cellulosefasern, optional aus versteiften Cellulosefasern, optional aus synthetischen Fasern (z.B. Polyolefinen) sowie aus superabsorbierenden Polymeren und anschließender Rückfaltung oder Übereinanderlegen erreicht werden.

**Prüfmethode**

Die nachfolgenden Tests werden, sofern nicht anders angegeben, mit Polymerisaten mit einer Teilchengröße von 300 - 600 μm (bestimmt nach der Siebmethode) durchgeführt.

Anti-caking Test:

5 g ± 0,1 Polymerisat mit einer Teilchengröße von 150 bis 850 μm werden in eine Wägeschale (57 mm) aus Aluminium eingewogen und homogen über die gesamte Schale verteilt. Die Schale wird gewogen. Anschließend wird die Schale für 3, 6 oder 24 h in einen Klimaschrank bei einer Temperatur von 35 °C und einer relativen Luftfeuchtigkeit von 80 % gestellt. Danach wird die Schale erneut gewogen. Eine weitere Wägeschale wird ausgewogen und ein Sieb mit einer Maschenweite von 1,5 mm darüber gestellt. Die Probe wird auf das Sieb gekippt.

Nach mehrmaligen leichten Klopfen auf das Sieb wird das Gewicht des durch das Sieb gefallenen Produkts bestimmt.

Der Test gilt als bestanden, wenn mehr als 90 Gew.-% des Produkts durch das Sieb gefallen sind. Es wird außerdem die Wasseraufnahme des Produktes bestimmt.

Methode zur Messung Oberflächenspannung:

Messung der Oberflächenspannung von wässrigen Lösungen mit dem Stalagmometer von Traube-Gerhardt.

Bei dem Stalagmometer handelt es sich um eine Art Vollpipette, deren Auslauf zu einer sorgfältig hergestellten Abtropffläche ausgestaltet ist. An dieser polierten Fläche bilden sich nacheinander Tropfen aus, deren Größe von der Oberflächenspannung des untersuchten Produktes abhängt. Je größer diese ist, um so größer auch der Tropfen und umgekehrt. Das Volumen der Pipette ist durch Ringmarken begrenzt. Da das Volumen konstant ist und die Größe der Tropfen von der Größe der Oberflächenspannung, ist die Zahl der Tropfen ein direk-tes Maß für die Oberflächenspannung. Der gefundene Wert wird dann der Tropfenzahl von reinem Wasser gegenübergestellt, dessen Oberflächenspannung bekannt ist.

150 g 0,9%ige NaCl-Lösung werden in einem 250 ml Becherglas vorgelegt und mit einem Magnetrührer (200 U/min) gerührt. 1 g des zu prüfenden Polymerisates wird langsam in die sich bildende Trombe der 0,9%igen NaCl-Lösung gestreut. Nach beendetem Einstreuen wird die Lösung 3 Minuten gerührt. Anschließend lässt man die Lösung 15 Minuten stehen.

Mit Hilfe eines Peleusballes wird die zu prüfende Lösung deutlich über die obere, das Pipettenvolumen begrenzende, Ringmarke gesaugt. Es wird die Tropfenanzahl zwischen der oberen und unteren Ringmarke ermittelt. Von jeder Lösung erfolgt eine Doppelbestimmung.

$$\text{Berechnung der Oberflächenspannung in mN/m} = \text{Tropfenzahl für reines Wasser} \times 72{,}75^* / \text{gefundene Tropfenzahl der Probe}$$

(Oberflächenspannung von Wasser in mN/m)

Die Messung der Oberflächenspannung dient zur Feststellung, wieviel Beschichtungsmittel gegf. an eine wässrige Umgebung abgegeben wird. D. h. umgekehrt durch diese Messung wird festgestellt, wie gut das Beschichtungsmittel mit dem Polymerisat verbunden ist. Wird nämlich die Oberflächenspannung durch die Beschichtung herabgesetzt kann verstärkt das Problem des "rewetten" auftreten. Unter Rewetten oder Rücknässen wird die Wiederabgabe von bereits absorbierter Flüssigkeit (z. B. Urin) z. B. bei Druckbelastung aus dem gequollenen Gel verstanden, wodurch der Tragekomfort eines Hygieneartikels beeinträchtigt wird.

Retention:

Die Retention wird nach der Teebeutelmethode und als Mittelwert aus drei Messungen angegeben. Etwa 200 mg Polymerisat werden in einen Teebeutel eingeschweißt und für 30 Minuten in 0,9%ige NaCl-Lösung getaucht. Anschließend wird der Teebeutel in einer Schleuder (23 cm Durchmesser, 1.400 Upm) 3 Minuten geschleudert und gewogen. Einen Teebeutel ohne absorbierendes Polymerisat lässt man als Blindwert mitlaufen.

$$\text{Retention [g/g]} = \text{Auswaage - Blindwert / Einwaage}$$

Flüssigkeitsaufnahme unter einem Druck von 0.9 psi AUL:

In einen Zylinder aus Plexiglas mit einem inneren Durchmesser von 25,4 mm, der mit einem 400 mesh Nylon-Siebboden ausgestattet ist, werden ca. 0,16 g Polymerisat genau eingewogen. Die gleichmäßig auf dem Siebboden verteilte Lage von Polymerisat wird mit einer Teflonscheibe von 26,1 mm Durchmesser abgedeckt und mit einem zylindrischen Stempel von 332,4 g belastet. Teflonscheibe und Zylinder bewirken zusammen eine Belastung von 63 g/cm$^2$ bzw. 0,9 psi. Der zuvor gewogene Zylinder wird anschließend auf eine Glasfilterplatte gestellt, die sich in einer Schale mit 0,9%iger NaCl-Lösung befindet, deren Flüssigkeitsniveau genau der Höhe der Filterplatte entspricht. Nachdem man die Zylindereinheit 1 Stunde lang 0,9%ige NaCl-Lösung hat saugen lassen, wird diese durch Abtupfen mit Filterpapier von überschüssiger Prüflösung befreit und zurückgewogen und der AUL wie folgt berechnet:

$$\text{AUL} = \text{Auswaage (Zylindereinheit + gequollenes Polymerisat) -Einwaage (Zylindereinheit + nichtgequollenes Polymerisat) / Einwaage Polymerisat}$$

**Beispiele**

Im folgenden wird die Erfindung anhand von Beispielen erläutert. Diese Erläuterungen sind lediglich beispielhaft und schränken den allgemeinen Erfindungsgedanken nicht ein.

Dabei werden folgende Abkürzungen verwendet:

| | |
|---|---|
| ABAH | 2,2'-Azo-bis-amidinopropan-dihydrochlorid |
| AIBN | 2,2'-Azo-bis-2-methylpropionitril |
| AMPS | 2-Acrylamido-2-methylpropansulfonsäure |
| BO | 2-Butyl-octanol |

| | |
|---|---|
| EO | Ethylenoxid (1,2-Epoxiethan) |
| IHD | Isohexadecan |
| ITDA | Isotridecylalkohol |
| ITS | Isotridecylstearat |
| NG | Neutralisationsgrad in Mol-% |
| ÖFSBOE | Ölfettsäurebutyloctylester |
| RÖFSME | Rübölfettsäuremethylester |
| TAMAC | Triallylmethylammoniumchlorid |

Vergleichsbeispiel 1 : US 5,728,742

Zu 50 g Polyacrylat Pulver, das zu 70% neutralisiert und oberflächennachvernetzt ist (Favor SXM 880 ®, ein Handelsprodukt von Stockhausen GmbH & Co. KG), mit einer Retention von 32 g/g in 0,9%iger NaCl-Lösung und einer $AUL_{0,9\ psi}$ 22.1 g/g wurden unter Rühren mit einem Mixer mittels einer Spritze 1000 ppm Ethoquad 0/12 in 3 g Isopropanol gelöst zugegeben. Das Polymerisat wurde für 60 Minuten auf einer Rollbank bei Raumtemperatur gerollt. Das Produkt bestand den vorstehend beschriebenen 3 h Anti-caking Test nicht.
Ethoquad 0/12 = Oleylmethyldi(2-hydroxyethyl)ammoniumchlorid

Vergleichsbeispiel 2 : US 5,728,742

Zu 50 g Polyacrylatpulver gemäß Vergleichsbeispiel 1 (Favor SXM 880 ®, ein Handelsprodukt von Stockhausen GmbH & Co. KG) wurden unter Rühren mit einem Mixer mittels einer Spritze 1000 ppm Arquad 16-50 in 3 g Isopropanol gelöst zugegeben. Das Polymerisat wurde für 60 Minuten auf einer Rollbank bei Raumtemperatur gerollt. Das Produkt bestand den vorstehend beschriebenen 3 h Anti-caking Test nicht.
Arquad 16-50 = Hexadecyltrimethylammoniumchlorid

Herstellung von Polymerisatpulvern 1 - 5

Pulver 1:

290 g Acrylsäure wurden in zwei gleiche Portionen geteilt. Eine Portion wurde in 458,5 g $H_2O$ gegeben. 0,85 g Polyethylenglykol-300-diacrylat und 1,5 g Allyloxypolyethylenglykolacrylsäureester wurden in der zweiten Portion Acrylsäure gelöst und ebenfalls dem Wasser hinzugefügt. Die Lösung wurde auf 10°C gekühlt. Danach wurden unter Kühlen insgesamt 225,4 g 50%ige Natronlauge so langsam zugesetzt, dass die Temperatur nicht 30°C überstieg. Anschließend wurde die Lösung bei 20°C mit Stickstoff gespült und dabei weiter abgekühlt. Nach Erreichung der Starttemperatur von 4°C wurden die Initiatorlösungen (0,1 g 2,2'-Azobis-2-amidinopropan-dihydrochlorid in 10 g $H_2O$, 1,0 g Natriumperoxodisulfat in 10 g $H_2O$, 0,1 g 30%ige Wasserstoffperoxidlösung in 1 g $H_2O$ und 0,015 g Ascorbinsäure in 2 g Wasser) zugesetzt. Nachdem die Endtemperatur 102°C erreicht war, wurde das entstandene Gel zerkleinert und bei 150°C 90 Minuten lang getrocknet. Das getrocknete Polymerisat wurde grob zerstoßen, gemahlen und auf ein Pulver mit einer Partikelgröße von 150 bis 850 µm gesiebt.

Pulver 2:

300 g Acrylsäure wurden in zwei gleiche Portionen geteilt. Eine Portion wurde in 429,1 g $H_2O$ gegeben. 0,36 g Triallylamin, 1,05 g Allyloxypolyethylenglykolacrylsäureester und 12 g Methoxypolyethylenglykol(22EO)methacrylat wurden in der zweiten Portion Acrylsäure gelöst und ebenfalls dem Wasser hinzugefügt. Die Lösung wurde auf 10°C gekühlt. Danach wurden unter Kühlen insgesamt 233,1 g 50%e Natronlauge so langsam zugesetzt, dass die Temperatur nicht 30°C überstieg. Anschließend wurde die Lösung bei 20°C mit Stickstoff gespült und dabei weiter abgekühlt. Bei Erreichen der Starttemperatur von 4°C wurden 0,9 g Natriumcarbonat und die Initiatorlösungen (0,1 g 2,2'-Azobis-2-amidinopropandihydrochlorid in 10 g $H_2O$, 0,15 g Natriumperoxodisulfat in 10 g $H_2O$, 0,1 g 30%ige Wasserstoffperoxidlösung in 1g $H_2O$ und 0,01 g Ascorbinsäure in 2 g Wasser) zugesetzt. Nachdem die Endtemperatur von 104°C erreicht

war, wurde das entstandene Gel zerkleinert und bei 150°C 90 Minuten getrocknet. Das getrocknete Polymerisat wurde grob zerstoßen, gemahlen und zu einem Pulver mit einer Partikelgröße von 150 bis 850 µm abgesiebt.

Pulver 3:

war eine nicht oberflächennachvernetzte Polyacrylsäure (Vorprodukt zu Favor SXM 880®), die zu 70% neutralisiert war und eine Retention von 40 g/g in 0,9 %iger NaCl-Lösung und einen AUL von 8,7 g/g aufwies.

Pulver 4:

280 g Acrylsäure wurden in zwei gleiche Portionen geteilt. Eine Portion wurde in 517,04 g $H_2O$ gegeben. 0,28 g Triallylamin, 0,72 g Allyloxypolyethylenglykolacrylsäureester und 7,51 g Methoxypolyethylenglykol(22EO)methacrylat wurden in der zweiten Portion Acrylsäure gelöst und ebenfalls dem Wasser hinzugefügt. Die Lösung wurde auf 10°C gekühlt. Danach wurden unter Kühlen insgesamt 170,97 g 50%ige Natronlauge so langsam zugesetzt, dass die Temperatur nicht über 30°C stieg. Anschließend wurde die Lösung bei 20°C mit Stickstoff gespült und dabei weiter abgekühlt. Nach Erreichung der Starttemperatur von 4°C wurden 0,9 g Natriumcarbonat und die Initiatorlösungen (0,1 g 2,2'-Azobis-2-amidinopropandihydrochlorid in 10 g $H_2O$, 1,0 g Natriumperoxodisulfat in 10 g $H_2O$, 0,07 g 30%ige Wasserstoffperoxidlösung in 1 g $H_2O$ und 0,01 g Ascorbinsäure in 2 g Wasser) zugesetzt. Nachdem die Endtemperatur 102°C erreicht war, wurde das entstandene Gel zerkleinert und bei 150°C 90 Minuten getrocknet. Das getrocknete Polymerisat wurde grob zerstoßen, gemahlen und zu einem Pulver mit einer Partikelgröße von 150 bis 850 µm gesiebt.

Pulver 5:

war das oberflächennachvernetzte Polyacrylat gemäß Vergleichsbeispiel 1 mit einer Retention von 32 g/g in 0,9 %iger NaCl-Lösung und einem AUL von 22,1 g/g (Favor SXM 880®).

Beispiel 1

Zu 50 g des Pulvers 1 wurde unter Rühren mit einem Mixer (Krups Dry Mix Typ 7007) mit höchster Drehzahlstufe eine Mischung aus 0,015 g Comperlan COD, 0,25 g Ethylencarbonat als Nachvernetzungsmittel 1,0 g $H_2O$ und 4,0 g Aceton zugegeben. Das beschichtete Polymerisat wurde in einer Photoschale gleichmäßig verteilt und 30 Minuten lang bei 180°C in einem Umlufttrockenschrank getrocknet. Von dem Polymerisat wurden die Retention, der AUL-Wert, die Oberflächenspannung und der Anti-caking Test, wie oben angegeben, durchgeführt und in der Tabelle 1 wiedergegeben.

Beispiele 2-17

Die Beispiele 2 - 17 wurden gemäß Beispiel 1 durchgeführt. Das eingesetzte Pulver, zugesetzten Verbindungen, die Menge der jeweiligen Verbindung sowie die Zeit und Temperaturen der Wärmebehandlung sind in der Tabelle 1 ebenso wie die entsprechenden Retentions-, AUL-, Oberflächenspannungs-Werte und die Ergebnisse des Anti-caking Tests aufgeführt.

Vergleichsbeispiel 3

Zu 50 g des Pulvers 5 wurde unter Rühren mit einem Mixer (Krups Dry Mix Typ 7007) mit höchster Drehzahlstufe eine Mischung aus 0,05 g Imbentin CMEA/045 in 1,0 g $H_2O$ zugegeben und 2 Minuten weitergerührt. Es wurde keiner Wärmebehandlung durchgeführt. Das Polymerisat bestand den 3 h Anti-caking Test nicht und hatte eine verminderte Rieselfähigkeit.

Das Polymerisat hatte eine Retention von 31,4 g/g und einen AUL-Wert von 21,3 g/g.

Beispiel 18

Zu 50 g des Pulvers 5 wurde unter Rühren mit einem Mixer (Krups Dry Mix Typ 7007) mit höchster Drehzahlstufe eine Mischung aus 0,05 g Imbentin CMEA/045 in 1,0 g $H_2O$ zugegeben und 2 Minuten weitergerührt. Das Produkt wurde in einer Photoschale verteilt und 20 Minuten bei 180°C in einem Umlufttrockenschrank getrocknet. Das behandelte Polymerisat bestand den Anti-caking Test. Seine Retentions-, AUL- und Oberflächenspannungs-Werte sind in Tabelle 1 aufgeführt.

Beispiel 19

Zu 50 g des Pulvers 5 wurde unter Rühren mit einem Mixer (Krups Dry Mix Typ 7007) mit höchster Drehzahlstufe eine Mischung aus 0,05 g Imbentin CMEA/045 in 1,0 g $H_2O$ zugegeben und 2 Minuten weitergerührt. Das Produkt wurde in einer Photoschale verteilt und 15 Minuten bei 190°C in einem Umlufttrockenschrank getrocknet. Das behandelte Polymerisat bestand den 3 h Anti-caking. Seine Retentions-, AUL- und Oberflächenspannungs-Werte sind in Tabelle 1 aufgeführt.

Tabelle 1

| Beispiel | Pulver | Anti-caking Substanz | Konzentration Gew% bezogen auf produkt | Temperatur/Zeit [°C/t] | Anti-caking Test 3 h | Retention [g/g] | AUL 0.9 psi [g/g] | Oberflächenspannung [mN/m] |
|---|---|---|---|---|---|---|---|---|
| | 1 | - | | 180/30 | - | 31,5 | 17,7 | |
| 1 | 1 | Comperlan COD | 0,015 | 180/30 | + | 31,5 | 17,6 | 72,5 |
| 2 | 1 | Comperlan LD | 0,06 | 180/30 | + | 32,8 | 17,4 | 72,5 |
| | 2 | - | | 180/30 | - | 34,8 | 23,5 | - |
| 3 | 2 | Marlazin L10 | 0,03 | 180/40 | + | 35,0 | 23,7 | 72,5 |
| 4 | 2 | Marlazin L10 | 0,10 | 180/40 | + | 34,9 | 23,8 | 72,5 |
| 5 | 2 | Serdox NXC 3 | 0,10 | 180/30 | + | 34,5 | 22,5 | 72,5 |
| 6 | 2 | Serdox NXC 6 | 0,10 | 180/40 | + | 35,0 | 21,0 | 72,5 |
| 7 | 2 | Serdox NXC 14 | 0,10 | 180/40 | + | 34,8 | 22,0 | 72,5 |
| 8 | 2 | Serdox NXC6 | 0,05 | 180/40 | + | 35,2 | 22,3 | 72,5 |
| | 3 | - | | 190/25 | - | 32,4 | 21,2 | - |
| 9 | 3 | Serdox NXC 6 | 0,10 | 180/35 | + | 32,3 | 19,1 | 72,5 |
| 10 | 3 | Marlazin OL 20 | 0,10 | 180/35 | + | 32,3 | 20,3 | 72,5 |
| 11 | 3 | Marlazin L 10 | 0,10 | 180/35 | + | 32,3 | 19,5 | 72,5 |
| 12 | 3 | Comperl an COD | 0,2 | 180/30 | + | 31,3 | 21,1 | 72,5 |
| 13 | 3 | Serdox NXC 6 | 0,1 | 180/30 | + | 31,7 | 21,2 | 72,5 |
| 14 | 3 | Imbentin CMEA/ 045 | 0,1 | 190/20 | + | 32,1 | 21,0 | 72,5 |
| 15 | 3 | Imbentin CMEA/ 045 | 0,2 | 190/20 | + | 32,0 | 20,8 | 72,5 |
| | 4 | - | | 170/25 | - | 30,5 | 21,0 | - |
| 16 | 4 | Stokomin S10 | 0,1 | 170/25 | + | 30,1 | 21,2 | 72,5 |
| 17 | 4 | Serdox NXC 3 | 0,1 | 170/25 | + | 30,9 | 20,4 | 72.5 |
| | 5 | - | | | | 32,0 | 22,1 | |

Tabelle fortgesetzt

| Beispiel | Pulver | Anti-caking Substanz | Konzentration Gew% bezogen auf produkt | Temperatur/Zeit [°C/t] | Anti-caking Test 3 h | Retention [g/g] | AUL 0.9 psi [g/g] | Oberflächenspannung [mN/m] |
|---|---|---|---|---|---|---|---|---|
| 18 | 5 | Imbentin CMEA/ 045 | 0,05 | 180/20 | + | 30,9 | 20,8 | 72,5 |
| 19 | 5 | Imbentin CMEA/ 045 | 0,05 | 190/15 | + | 30,8 | 21,0 | 72,5 |

Stokomin S10 = Stearylamin, ethoxyliert mit 10 EO; Comperlan COD = Kokossäurediethanolamid (Henkel KGaA); Comperlan LD = Laurylsäurediethanolamid (Henkel KGaA); Comperlan 100 = Kokossäuremonoethanolamid (Henkel KGaA); Marlazin L 10 = Laurylamin, ethoxyliert mit 10 EO (Contensio); Marlazin OL 20 = Oleylamin, ethoxyliert mit 20 EO (Contensio); Serdox NXC 3 = Olsäuremonoethanolamid, ethoxyliert mit 3 EO (Condea); Serdox NXC 6 = Olsäuremonoethanolamid, ethoxyliert mit 6 EO (Condea); Serdox NXC 14 = Olsäuremonoethanolamid, ethoxyliert mit 14 EO (Condea); Imbentin CMEA/045 = Kokossäuremonoethanolamid, ethoxyliert mit 4,5 EO

Vergleichswert der Oberflächenspannung für Wasser: $\sigma$ = 72,5 mN/m

+ bedeutet Test bestanden

- bedeutet Test nicht bestanden

Vergleichsbeispiel 4

0,5 g Ethylencarbonat, 2 g Wasser und 0,25 g $Ab(SO_4)_3$ x $18H_2O$ wurden gemischt und zu 50 g des Pulvers 3 unter Rühren mit dem Mixer (Krups Dry Mix Typ 7007) mit höchster Drehzahlstufe mittels einer Spritze zugegeben. Anschließend wird das Produkt in einer Photoschale verteilt und 50 min bei 170°C im Umlufttrockenschrank getrocknet.
Das behandelte Polymerisat bestand den 3h Anti-caking Test nicht. Seine Retentions-, AUL-Werte sind in Tabelle 2 aufgeführt.

Beispiel 20

Das Pulver 3 wurde mit 0,7 Gew.% Ethylencarbonat, 1,8 Gew-% Wasser, 0,2 Gew.% $Al_2(SO_4)_3$ x $14H_2O$ und 0,2 Gew.% Imbentin CMEA/024 (Firma Kolb AG) in einem Mixer (Krups Dry Mix Typ 7007) mit höchster Drehzahlstufe gemischt und so beschichtet. Anschließend wird das behandelte Polymerisat in einen Schaufeltrockner gefördert und dort 10 min bei einer Temperatur von 110°C belassen.
Das Produkt bestand den 3h Anti-caking Test. Seine Retentions-, AUL-Werte sind in Tabelle 2 aufgeführt.

Beispiel 21

0,5 g Ethylencarbonat, 0,5 g Aceton, 2 g Wasser, 0,075 g $ZnCl_2$ und 0,05 g Imbentin CMEA/045 (Firma Kolb AG) werden gemischt und zu 50 g des Pulvers 3 unter Rühren mit dem Mixer (Krups Dry Mix Typ 7007) mit höchster Drehzahlstufe mittels einer Spritze zugegeben. Anschließend wurde das Polymerisat in einer Photoschale verteilt und 50 min lang bei 170°C im Umlufttrockenschrank getrocknet.
Das behandelte Polymerisat bestand den 3h Anti-caking Test. Seine Retentions-, AUL- und Oberflächenspannungs-Werte sind in Tabelle 2 aufgeführt.

Beispiel 22

Das Pulver 3 wurde durch Zugabe einer Mischung aus 2 Gew.% Wasser und 1 Gew.% $H_2SO_4$ (98%ig) unter Rühren mit dem Mixer mittels einer Spritze beschichtet. Dann wurden 0,5 g Ethylencarbonat, 0,5 g Aceton, 0,05 g Imbentin CMEA/045 und 1 g Wasser gemischt und zu 50 g des Pulvers 3 unter Rühren mit dem Mixer (Krups Dry Mix Typ 7007) mit höchster Drehzahlstufe mittels einer Spritze gegeben. Anschließend wird das Polymerisat in einer Photoschale verteilt und 50 min lang bei 170°C im Umlufttrockenschrank getrocknet.
Das behandelte Polymerisat bestand den 3h Anti-caking Test. Seine Retentions-, AUL- und Oberflächenspannungs-Werte sind in Tabelle 2 aufgeführt.

Beispiel 23

0,5 g Ethylencarbonat, 0,5 g Aceton, 1 g Wasser, 1 g $H_3PO_4$ (85%ig) und 0,1 g Imbentin CMEA/045 (Firma Kolb AG) wurden gemischt und zu 50 g des Pulvers 3 unter Rühren mit dem Mixer (Krups Dry Mix Typ 7007) mit höchster Drehzahlstufe mittels einer Spritze beschichtet. Anschließend wurde das Polymerisat in einer Photoschale verteilt und 50 min lang bei 170°C im Umlufttrockenschrank getrocknet.
Das behandelte Polymerisat bestand den 3h Anti-caking Test. Seine Retentions-, AUL- und Oberflächenspannungs-Werte sind in Tabelle 2 aufgeführt.

Beispiele 24 - 27

Die Beispiele 24 - 29 wurden gemäß Beispiel 21 durchgeführt, wobei die in Tabelle 2 angegebenen Lewissäuren und Imbentin CMEA/045 in den dort angegebenen Mengen verwendet wurden. Die Resultate des 3 h Anti-caking Tests, die Retentions-, AUL- und Oberflächenspannungs-Werte der jeweiligen Polymeren sind in Tabelle 2 aufgeführt.

**Tabelle 2**

| Beispiel | Lewis-Säure | Imbentin* Lewis-Säure** (Gew.%) | / Temp. / Zeit (°C/min) | Retention (g/g) | $AUL_{0.9\ psi}$ (g/g) | Anti-caking 3h |
|---|---|---|---|---|---|---|
| Vergl. beisp. 4 | $Al_2(SO_4)_3$ x $18H_2O$ | 0,0/0,50 | 170/50 | 31,5 | 22,6 | - |
| 20 | $Al_2(SO_4)_3$ x $14H_2O$ | 0,2 / 0,20 | 110/10 | 35,7 | 7,2 | + |
| 21 | $ZnCl_2$ | 0,1/0,15 | 170/50 | 32,8 | 21,8 | + |
| 22 | $H_2SO_4$ | 0,1/1,00 | 170/50 | 31,9 | 19,0 | + |
| 23 | $H_3PO_4$ | 0,2/2,00 | 170/50 | 30,0 | 17,1 | + |
| 24 | $FeCl_3x6H_2O$ | 0,1/0,05 | 170/50 | 32,3 | 21,4 | + |
| 25 | $MgSO_4x7H_2O$ | 0,3/0,10 | 170/50 | 31,5 | 23,6 | + |
| 26 | $AlCl_3x6H_2O$ | 0,1/0,05 | 160/50 | 35,9 | 14,0 | + |
| 27 | $FeCl_36H_2O$ | 0,2/0,10 | 170/50 | 32,1 | 19,2 | + |

* Imbentin CMEA/045
** bezogen auf Komponente I
+ bedeutet Anti-caking Test bestanden
- bedeutet Anti-caking Test nicht bestanden

Beispiele 28 - 29

0,5g Ethylencarbonat, 2 g Wasser, Aluminiumsulfat und Imbentin CMEA/045 (Firma Kolb AG) werden gemischt und zu 50 g des Pulvers 3 unter Rühren mit dem Mixer (Krups Dry Mix Typ 7007) mit höchster Drehzahlstufe mittels einer Spritze zugegeben. Anschließend wurde das Polymerisat in einer Photoschale verteilt und 60 min lang bei 170 °C im Umluftrockenschrank getrocknet. Reaktionsbedingungen und Eigenschaften der Produkte sind in Tabelle 3 zusammengefasst.

Beispiele 30 - 33

Entsprechend der Herstellung des Pulvers 1 werden zwei Polyacrylsäuren hergestellt mit einem Neutralisationsgrad von 65 bzw. 70 % und einer Retention von 34 bzw. 32 g/g in 0,9 %iger wässriger Nacl-Lösung, 50 g dieser Pulver (Teilchengröße in einem Bereich von 150 bis 850 µm) wurden mit einer Lösung bestehend aus 0,5 g Ethylencarbonat, 2 g Wasser, Aluminiumsulfat x 14 $H_2O$ und Imbentin CMEA/045 mittels eines Mixers (Krups Dry Mix Typ 7007) mit höchster Drehzahlstufe vermischt. Anschließend wurde das Pulver in einer Photoschale im Umlufttrockenschrank getrocknet. Reaktionsbedingungen und Eigenschaften der Produkte sind in Tabelle 3 zusammengefasst.

Tabelle 3

| Bsp. | Ausgangsprodukt produkt NG | Retention g/g | Imbentin[1] | $Al_2(SO_4)_3 x 14H_2O$[1] | Temp. °C | Zeit Min | Anti-caking Test[2] | Retention | AUL 0,9 psi g/g |
|---|---|---|---|---|---|---|---|---|---|
| 28 | 70 | 39 | 0,1 | 0,1 | 170 | 60 | 6 | 31,7 | 22,0 |
| 29 | 70 | 39 | 0,1 | 0,2 | 170 | 60 | 6 | 31,6 | 21,5 |
| 30 | 65 | 34 | 0,25 | 0,45 | 170 | 60 | 24 | 30 | 20,5 |
| 31 | 65 | 34 | 0,25 | 0,50 | 170 | 60 | 24 | 30 | 21 |
| 32 | 70 | 32 | 0,30 | 0,50 | 180 | 30 | 24 | 28 | 19,5 |
| 33 | 70 | 32 | 0,25 | 0,60 | 180 | 30 | 24 | 30 | 20 |

[1] Gew.-% bezogen auf das Pulver

[2] Anticaking Test bestanden nach 6 bzw. 24 Stunden

## Patentansprüche

1. Ein hochquellfähiges Absorptionsmittel mit einer verminderten Tendenz zum Verbacken an feuchter Umgebung und/oder bei hohen Temperaturen, mindestens basierend auf Komponenten

   I einem mit Säuregruppen modifizierten, Wasser oder wässrige Flüssigkeiten absorbierenden natürlichen Polymeren oder einem wassetunlöslichen, an der Oberfläche nachvernetzten, Wasser oder wässrige Flüssigkeiten absorbierenden, vernetzten Polymerisat basierend auf polymerisierten, mindestens teilneutralisierten. Säuregruppen enthaltenden Monomeren, das mit
   II wenigstens einem Beschichtungsmittel aus der Gruppe Stickstoffhaltiger, nichtionischer Tenside behandelt und

   die Mischung aus der Komponente I und der Komponente II
   einer Wärmebehandlung unterworfen worden ist.

2. Das Absorphonsmittel nach Anspruch 1, wobei die Komponente I auf (Meth)acrylsäure basiert, deren Carboxylgruppen zu mindestens 50 Mol% neutralisiert sind.

3. Ein hochquellfähiges Absorptionsmittel nach Anspruch 1 oder 2, welches nach mindestens drei Stunden Wärmebehandlung gemäß des in der Beschreibung beschriebenen Anticaking Tests nicht verbackt und welches die folgenden Eigenschaften aufweist

   a) eine Retention von mindestens 20 g/g; und
   b) eine Absorption under Load bei einer Belastung von 0,9 psi ($AUL_{0.9psi}$) von mindestens 7 g/g.

4. Ein Verfahren zur Herstellung eines Absorptionsmittel nach einem der vorhergehenden Ansprüche, wobei man als Komponente I ein Wasser oder wässrige Flüssigkeiten absorbierendes, mit Säuregruppen modifiziertes, natürliches Polymeres oder ein Wasser oder wässrige Flüssigkeiten absorbierendes, vernetztes Polymerisat basierend auf polymerisierten, mindestens teilneutralisierten Säuregruppen enthaltenen Monomeren mit einem Beschichtungsmittel aus der Gruppe stickstoffhaltige, nicht ionische Tenside als Komponente II und ggf. eine Lewissäure als Komponente III beschichtet und die Mischung einer Wärmebehandlung unterwirft.

5. Ein Absorptionsmittel erhältlich nach einem Verfahren gemäß Anspruch 4.

6. Verwendung der Absorptionsmittel nach Anspruch 1 bis 3 oder 5 in Composites zur Absorption von Wasser, wässrige oder seröse Flüssigkeiten, vorzugsweise Körperflüssigkeiten.

7. Verwendung der Absorptionsmittel nach Anspruch 1 bis 3 oder 5 in Hygieneartikeln, vorzugsweise, Windeln, Tampons oder Damenbinden.

8. Verwendung der Absorptionsmittel nach Anspruch 1 bis 3 oder 5 in Kabelummantelungen oder Verpackungen.

9. Verwendung der Absorptionsmittel nach Anspruch 1 bis 3 oder 5 als Wasserspeicher in Böden oder Substraten.

10. Verwendung der Absorptionsmittel nach Anspruch 1 bis 3 oder 5 als Speicher für Nähr- und Wirkstoffe und deren kontrollierter Abgabe.

11. Windeln, Inkontinenzprodukte für Erwachsene, Damenhygieneartikel, enthaltend Absorptionsmittel vorzugsweise in Composites, gemäß einer der Ansprüche 1 bis 3 oder 5.

## Claims

1. A highly swellable absorption agent with a reduced caking tendency in a moist environment and/or at high temperatures based at least on the following components:

   I a water- or aqueous fluid-absorbing natural polymer modified with acid groups or a water-insoluble, optionally surface cross-linked, water- or aqueous fluid-absorbing cross-linked polymer based on polymerized monomers

containing at least partially neutralized acid groups, which is treated with:
II at least one coating agent selected from the group formed by nitrogen-containing, non-ionic surfactants; and whereby the mixture formed from components I and II has been heat treated.

**2.** The absorption agent according to claim 1, wherein the component I is based on (meth)acrylic acid, the carboxyl groups of which are at least 50 mole% neutralized.

**3.** A highly swellable absorption agent according to claim 1 or 2, which does not cake after at least three hours of heat treatment according to the anticaking test described in the description and which has the following characteristics:

a) a retention of at least 20 g/g; and
b) an absorption under load at a load of 0,9 psi ($AUL_{0,9psi}$) of at least 7 g/g.

**4.** A process for the production of an absorption agent according to one of the preceding claims, wherein component I is a water- or aqueous fluid-absorbing, natural polymer modified with acid groups of a water- or aqueous fluid-absorbing, cross-linked polymer based on polymerized monomers containing at least partially neutralized acid groups is coated with a coating agent selected from the group formed by nitrogen-containing, non-ionic surfactants as component II and optionally a Lewis acid as component III and the mixture is subjected to a heat treatment.

**5.** An absorption agent obtainable according to a process according to claim 4.

**6.** Use of the absorption agents according to claim 1 to 3 or 5 in composites for absorbing water, aqueous or serous liquids, preferably body liquids.

**7.** Use of the absorption agents according to claim 1 to 3 or 5 in hygiene articles, preferably nappies, tampons or feminine pads.

**8.** Use of the absorption agents according to claim 1 to 3 or 5 in cable sheaths or packaging.

**9.** Use of the absorption agents according to claim 1 to 3 or 5 as a water storage means in floors or substrates.

**10.** Use of the absorption agents according to claim 1 to 3 or 5 to store nutrients and active agents and for their controlled release.

**11.** Nappies, incontinence products for adults, feminine hygiene articles, containing absorption agents, preferably in composites, according to any one of claims 1 to 3 or 5.

## Revendications

**1.** Agent d'absorption hautement gonflable avec une tendance réduite à s'agglomérer dans un milieu humide et/ou à des températures élevées, au moins basant sur des composants :

I un polymère naturel modifié avec des groupes acides, absorbant de l'eau ou des liquides aqueux ou un polymérisat réticulé insoluble à l'eau, post-réticulé à la surface, absorbant de l'eau ou des liquides aqueux, basant sur des monomères polymérisés, contenant des groupes acides au moins partiellement neutralisés qui a été traité avec
II au moins un agent de revêtement, choisi parmi le groupe consistant en des surfactants non-ioniques contenant de l'azote,

le mélange du composant I et du composant II a été soumis à un traitement thermique.

**2.** Agent d'absorption selon la revendication 1, le composant I basant sur de l'acide (méth)acrylique dont les groupes carboxyles sont neutralisés à au moins 50 pourcent molaires.

**3.** Agent d'absorption hautement gonflable selon la revendication 1 ou 2 qui après au moins trois heures de traitement thermique n'agglomère pas selon le test d'anti-agglomération décrit dans la description et qui a les caractéristiques suivantes :

a) une rétention d'au moins 20 g/g ; et
b) une absorption sous charge à une charge de 0,9psi ($AUL_{0,9psi}$) d'au moins 7g/g.

**4.** Procédé pour la fabrication d'un agent d'absorption selon l'une des revendications précédentes, dans quel cas on revêtit comme composant I un polymère naturel absorbant de l'eau ou des liquides aqueux, modifié avec des groupes acides ou un polymérisat réticulé absorbant de l'eau ou des liquides aqueux basant sur des monomères polymérisés, contenant des groupes acides au moins partiellement neutralisés avec un agent de revêtement, choisi parmi le groupe consistant en des surfactants contenant de l'azote et non-ioniques comme composant II et le cas échéant un acide de Lewis comme composant III et on soumet le mélange à un traitement thermique.

**5.** Agent d'absorption qui peut être obtenu selon un procédé selon la revendication 4.

**6.** Utilisation des agents d'absorption selon la revendication 1 à 3 ou 5 dans des composites pour l'absorption d'eau, de liquides aqueux ou séreux, de préférence de liquides corporels.

**7.** Utilisation des agents d'absorption selon la revendication 1 à 3 ou 5 dans des articles hygiéniques, de préférence des couches, des tampons ou des serviettes hygiéniques.

**8.** Utilisation des agents d'absorption selon la revendication 1 à 3 ou 5 dans des enveloppes de câbles ou des emballages.

**9.** Utilisation des agents d'absorption selon la revendication 1 à 3 ou 5 comme réservoir d'eau dans les sols ou substrats.

**10.** Utilisation des agents d'absorption selon la revendication 1 à 3 ou 5 comme réservoir pour des substances nutritives et actives et leur décharge contrôlée.

**11.** Couches, produits d'incontinence pour adultes, articles hygiéniques pour femmes, contenant des agents d'absorption, de préférence en composites selon l'une des revendications 1 à 3 ou 5.